# FASCICULE DE BREVET EUROPEEN

(11) **EP 1 804 729 B1**
(45) Date de publication et mention de la délivrance du brevet: **12.08.2009**
(21) Numéro de dépôt: 05814983.2
(22) Date de dépôt: 25.10.2005
(51) Int. Cl.: A61F 2/40

(54) **PROTHESE HUMERALE MODULAIRE POUR PROTHESE D'EPAULE INVERSEE**
MODULARE OBERARMPROTHESE FÜR EINE INVERSE SCHULTERPROTHESE
MODULAR HUMERAL PROSTHESIS FOR AN INVERSE SHOULDER PROSTHESIS

(30) Priorité: 25.10.2004 FR 0411366
(43) Date de publication de la demande: 11.07.2007
(73) Titulaire: Depuy (Ireland) Limited, Ringaskiddy, County Cork (IE); Nerot, Cécile, 51100 Reims (FR); Capon, Didier, 44880 Sautron (FR); Seebauer, Ludwig, 85661 Forstinning (DE); Ekelund, Anders, 167 61 Bromma (SE); De Wilde, Lieven, 9000 Gent (BE); Wirth, Michael, San Antonio, TX 78232 (US); Collins, David, Little Rock, AR 72212 (US); LaFosse, Laurent, 74940 Annecy Le Vieux (FR)
(72) Inventeur: NEROT, Cécile, F-51100 Reims (FR); CAPON, Didier, F-44880 Sautron (FR); SEEBAUER, Ludwig, 85661 Forstinning (DE); EKELUND, Anders, S-167 61 Bromma (SE); DE WILDE, Lieven, B-9000 Gent (BE); WIRTH, Michael, San Antonio, TX 78232 (US); COLLINS, David, Little Rock, AR 72223 - 1317 (US); LAFOSSE, Laurent, F-74940 Annecy Le Vieux (FR); PONCET, Didier, F-69500 BRON (FR)
(74) Mandataire: Domenego, Bertrand
(86) Numéro de dépôt international: PCT/FR2005/002663
(87) Numéro de publication internationale: WO 2006/045949

(56) Documents cités:
- EP-A- 0 339 530
- EP-A- 0 679 375
- EP-A- 0 815 810
- EP-A- 1 402 854
- WO-A-97/25943
- DE-A- 19 841 612
- FR-A- 2 579 454
- FR-A- 2 699 400
- FR-A- 2 821 545
- US-A- 5 910 171
- US-A1- 2004 064 187
- US-B1- 6 790 234

## Description

La présente invention est relative à une prothèse humérale modulaire pour prothèse d'épaule inversée.

On connaît- des prothèses d'épaule constituées d'une prothèse humérale dont la partie épiphysaire coopère avec un composant deltoïdal complémentaire. En particulier, on connaît des prothèses d'épaule anatomiques dans lesquelles la tête de prothèse humérale est convexe. Ces prothèses respectent l'orientation et le centre de rotation des articulations réelles. Cependant, et notamment lorsque la coiffe des rotateurs est rompue (partiellement ou totalement), de telles prothèses ne permettent pas d'obtenir l'élévation du bras.

Afin de rétablir l'amplitude de rotation interne du bras (notamment l'élévation et l'abduction), on remplace la prothèse anatomique par une prothèse inversée dans laquelle la tête épiphysaire de la prothèse humérale est concave. Une telle prothèse inversée déplace le centre de rotation de l'épaule, ce qui augmente le bras de levier du muscle deltoïde et ainsi facilite l'élévation du bras. Cependant, ce déplacement du centre de rotation limite partiellement les rotations internes et externes du bras. Afin de privilégier la rotation interne (qui permet au patient de placer sa main dans le dos), la tête épiphysaire sera placée en rétroversion proche de 0° (plan frontal).

Outre le fait qu'elle déplace le centre de rotation de l'articulation, la prothèse d'épaule inversée modifie l'angle de rétroversion de la prothèse par rapport aux épaules. Cette modification de l'angle de rétroversion de la prothèse est variable d'un patient à l'autre. De ce fait, lorsqu'une prothèse d'épaule inversée est mise en place, il est nécessaire de l'orienter convenablement par rapport à l'anatomie propre du patient. De même, il est souhaitable que la prothèse inversée soit mise en place de façon telle que le déplacement du centre de rotation de l'épaule n'engendre pas une distension trop importante des tendons du muscle deltoïde.

Pour réaliser des prothèses d'épaule inversées, on a proposé d'utiliser une prothèse humérale monobloc dont la tige destinée à coopérer avec l'humérus est de révolution, et en particulier conique. Une telle prothèse humérale a l'avantage de pouvoir être orientée à volonté par rapport à l'humérus mais elle présente 'inconvénient de ne pas être bloquée en rotation par rapport à l'os, si bien qu'au fil du temps, la prothèse peut bouger et ainsi se dérégler.

Pour remédier à cet inconvénient, il a été proposé d'utiliser une prothèse humérale dont la tige présente une forme anatomique, c'est-à-dire une tige dont la section coopère avec celle de la partie métaphysaire du canal médullaire dans une position bien définie. Cependant, avec une telle tige humérale anatomique, il est nécessaire de pouvoir orienter la tête épiphysaire de la prothèse par rotation autour de l'axe longitudinal de la tige. Pour cela, on a proposé, par exemple, une tige humérale modulaire constituée d'une tige anatomique et d'une tête épiphysaire séparable montée sur la tige anatomique-par l'intermédiaire d'une rotule (brevet EP 1-402 805). Cette prothèse d'épaule inversée présente l'inconvénient d'avoir une tête épiphysaire qui s'étend très largement au dessus de l'épiphyse de l'humérus (en dehors de l'os huméral). Il en résulte une augmentation importante de la distance entre l'humérus et la glène, ce qui engendre une tension trop importante du muscle deltoïde.

Afin d'éviter de trop distendre les ligaments du muscle deltoïde, il a été proposé, notamment dans la demande brevet US-2004/064187, une tige humérale modulaire pour prothèse d'épaule inversée qui comporte une tige anatomique sur laquelle est montée une tête épiphysaire conçue pour s'intégrer à l'intérieur de l'épiphyse de l'humérus, et qui peut recevoir soit une tête humérale pour une articulation anatomique, soit une tête humérale complément d'articulation concave pour une prothèse inversée. La tête épiphysaire de cette prothèse peut être orientée par rotation autour de l'axe longitudinal de la tige humérale et être bloquée en positon par rapport à l'humérus par l'intermédiaire de nervures verticales prévues dans la tête épiphysaire et qui viennent coopérer avec la paroi interne du canal de l'humérus. Cette prothèse présente l'inconvénient de ne pas disposer de moyens permettant de régler facilement l'orientation de la tête épiphysaire par rapport à la tige anatomique. En effet, une des caractéristiques des prothèses d'épaule inversées munies d'une tige anatomique est que l'orientation de la tête épiphysaire par rapport à la tige anatomique, doit être adaptée pour chaque patient. Cette orientation, qui correspond à l'angle de rétroversion de l'articulation peut varier de 10° à 30° selon les patients, et en particulier selon leur age. Il est donc important de pouvoir adapter et maîtriser l'orientation de l'angle de la tête épiphysaire par rapport à la tige anatomique au cas par cas. En outre, lorsque la prothèse est en place, le réglage angulaire de la tête épiphysaire par rapport à la tige doit être verrouillé afin d'éviter tout déréglage au fil du temps.

Pour résoudre le problème de verrouillage en position de la tête épiphysaire par rapport à la tige, on pourrait utiliser des tiges humérales anatomiques monobloc. Mais, les dimensions de la tige anatomique doivent être adaptées à la taille du patient. Aussi, l'utilisation de tiges humérales anatomiques monobloc nécessiterait de disposer d'un nombre très important de tiges correspondant chacune à une taille et à un angle de rétroversion. De ce fait, pour réduire le nombre de prothèses nécessaire pour pouvoir répondre aux besoins de l'ensemble des patients, il est souhaitable de disposer de prothèses modulaires dans lesquelles on puisse associer une tête épiphysaire avec une tige anatomique de taille adaptée, et on puisse orienter avec précision la tête épiphysaire par rapport à la tige anatomique au moment de la pause de la prothèse et que cette orientation soit sécurisée.

Par FR 2 579 454, on connaît une prothèse humérale modulaire dont la tête épiphysaire peut être orientée par rapport à la tige anatomique. Mais, cette prothèse n'est pas adaptée à la réalisation d'une prothèse d'épaule inversée.

Outre ce problème de la réduction du nombre de pièces nécessaires pour répondre à l'ensemble des besoins, il est souhaitable que la prothèse puisse être extraite en cas de révision. De ce fait, la tige humérale (réglée angulairement par rapport à l'épiphyse) ne doit jamais dépasser latéralement de l'épiphyse afin de permettre l'extraction de l'implant.

Le but de la présente invention est de remédier aux inconvénients des prothèses connues en proposant une tige humérale modulaire pour prothèse d'épaule inversée, qui permette d'orienter avec précision et de façon sécurisée la tête épiphysaire par rapport à la partie métaphysaire de la tige anatomique (afin d'optimiser la rotation interne de l'humérus), qui puisse être extractible et qui, lorsqu'elle est en place, est complètement à l'intérieur de l'humérus de façon à ne pas créer de tension trop importante du deltoïde.

A cet effet, l'invention a pour objet une prothèse humérale modulaire du type comprenant une tige anatomique et une tête épiphysaire séparable pouvant être orientée angulairement par rotation autour de l'axe longitudinal de la tige anatomique, dans laquelle la tige anatomique et la tête épiphysaire comportent des moyens complémentaires de centrage, de guidage en rotation d'indexation angulaire et de blocage en rotation de l'un par rapport à l'autre, et pour rendre solidaire la tête épiphysaire et la tige anatomique, les moyens complémentaires comprenant une vis qui peut passer à travers des trous alignés prévus dans la tête éphiphysaire et dans la tige anatomique et qui s'étendent dans l'axe longitudinal de la tige pour connecter la tête épiphysaire à la tige, caractérisé en ce que :
a) la tête épiphysaire comprend une cuvette pour recevoir une cupule d'une prothèse d'épaule inversée coopérant avec une prothèse d'omoplate ;
b) la vis a une extrémité filetée et un fût entre la tête et l'extrémité filetée ;
c) le trou dans la tête épiphysaire, destiné à recevoir la vis, est fileté de sorte que l'extrémité filetée de la vis peut y être vissée et le fût peut y coulisser, et
d) le trou dans la tête épiphysaire s'ouvre dans la cuvette de sorte que la tête de la vis est située dans la cuvette lorsque la vis connecte la tête épiphysaire et la tige.

Les moyens complémentaires d'indexage et de blocage en rotation sont, par exemple, d'une part une pluralité d'encoches prévues dans une surface de contact de la tête épiphysaire avec la tige anatomique, disposées selon une répartition angulaire radiale autour de l'axe longitudinal de tige humérale, et d'autre part un plot porté par l'autre surface de contact de la tige anatomique avec la tête épiphysaire et pouvant coopérer avec lesdites encoches.

De préférence, la tige anatomique comprend au moins une nervure longitudinale de blocage en rotation.

De préférence, la fonction entre la tige anatomique et la tête épiphysaire est prévue pour se situer à l'intérieure de l'humérus lorsque la prothèse est en place, afin de respecter la tension du deltoïde. Il est alors préférable que la surface de contact de la tête épiphysaire s'étende latéralement au-delà de la tige anatomique.

L'invention va maintenant être décrite plus en détail mais de façon non limitative en regard des figures annexées dans lesquelles :
- La figure 1 représente une vue éclatée d'une prothèse humérale modulaire pour prothèse d'épaule inversée,

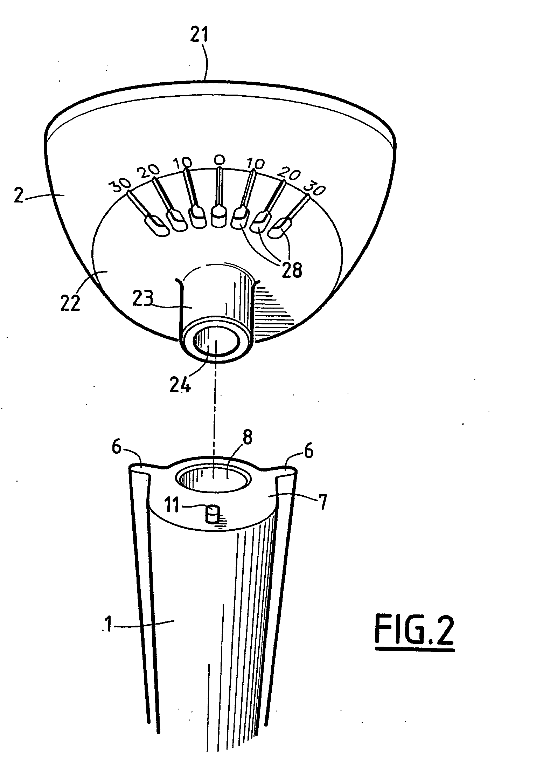

- La figure 2 est une vue agrandie et par en dessous de la tête épiphysaire et de la partie supérieure de la tige anatomique d'une prothèse humérale modulaire pour prothèse d'épaule inversée,
- La figure 3 est une vue éclatée en coupe schématique d'une prothèse humérale modulaire pour prothèse d'épaule inversée en position à l'intérieur d'un humérus.
- la figure 4 est une vue en coupe longitudinale agrandie de la partie supérieure de la prothèse humérale modulaire de la figure 3.

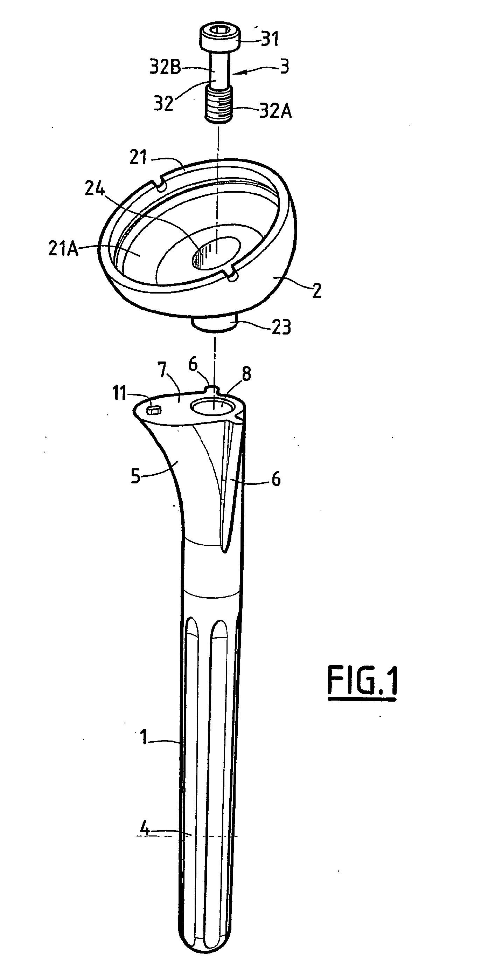

La prothèse humérale modulaire pour prothèse d'épaule inversée représentée à la figure 1, comporte une tige anatomique 1, une tête épiphysaire séparable 2 venant se positionner à l'extrémité supérieure (ou extrémité proximale) de la tige anatomique 1, et une vis 3 de liaison de la tête épiphysaire 2 avec la tige 1.

La tige anatomique de forme connue en elle-même, comporte un fût 4 généralement cylindrique qui se prolonge dans sa partie supérieure, par une partie métaphysaire 5 s'évasant vers le haut de façon à avoir une forme qui s'adapte à la forme de la jonction de l'épiphyse d'un humérus avec la métaphyse du même humérus. Cette tête 5 constitue l'extrémité proximale de la tige anatomique, la paroi latérale de la tête 5 de la tige anatomique comporte des nervures 6 de blocage en position par rapport à un humérus dans lequel la tige est implantée.

L'extrémité proximale de la tige anatomique 1 est limitée par une surface plane 7 qui correspond à une coupe perpendiculaire à l'axe longitudinal de la tige anatomique. Un trou 8 s'étendant à l'intérieur de la tige anatomique parallèlement à l'axe de cette tige est percé perpendiculairement à la surface 7 qui délimite l'extrémité proximale. Le trou 8 comporte un premier alésage 9 poursuivi par un trou fileté de plus petit diamètre 10. De préférence, le trou 8 et le fût 4 de la tige anatomique sont coaxiaux. La surface 7 comporte en outre un plot 11 en saillie longitudinal par rapport à la tige métaphysaire.

La tête épiphysaire 2, est une portion de sphère délimitée par un plan équatorial 21, et un plan formant avec le plan équatorial un angle aigu. En dessous du plan équatorial 21, la tête épiphysaire 2 comporte une cuvette 21A destinée à recevoir une cupule en polyéthylène ou en céramique. Cette cupule est la pièce destinée à coopérer avec la partie complémentaire de la prothèse qui sera fixée sur l'omoplate. Le plan formant avec le plan équatorial un angle aigu, définit une surface polaire 22 destinée à venir au contact de la surface d'extrémité 7 de la tige métaphysaire anatomique 1.

La surface polaire 22 comporte un plot cylindrique 23 en saillie par rapport à la surface polaire 22 et perpendiculaire à celle-ci. Ce plot cylindrique 23 est d'une taille adaptée pour pouvoir-coopérer avec l'alèsage de plus grand diamètre 9 du trou axial 8 de la tige anatomique et ainsi centrer la tête épiphysaire par rapport à la tige anatomique. Le plot cylindrique 23 est traversé par un trou axial 24 destiné à recevoir une vis 3 qui s'étendra à la fois dans le trou 24 de la tête épiphysaire et dans le trou 8 de la tige anatomique de façon à venir se visser dans la partie fileté 10 du trou 8. Le trou 24 comporte une première partie 25 de plus grand diamètre destinée à recevoir la tête 31 de la vis, et une partie de plus petit diamètre 26 destinée à recevoir le corps 32 de la vis 3. Le plot cylindrique 23 et le trou 8 constituent des moyens de centrage et de guidage en rotation de la tête épiphysaire par rapport à la tige anatomique.

La partie de plus petit diamètre 26, comporte une première partie 26A de faible longueur ayant un diamètre et un filetage identiques au diamètre et un filetage de la partie filetée 10 du trou axial 8, de la tige métaphysaire et une deuxième partie 26B de plus grande longueur, s'étendant jusqu'au débouché du trou à l'extrémité du plot cylindrique 23, et dont le diamètre est supérieur ou égal au diamètre extérieur de la partie fileté de la vis 3. Le corps 32 de la vis 3 comporte, à son extrémité, un bout fileté 32A pouvant coopérer avec le filetage de la partie filetée 10 du trou axial 8 de la tige métaphysaire, et un fût 32B reliant la tête 31 de la vis 3 au bout fileté, et de diamètre inférieur au diamètre intérieur de la première partie filetée 26A de la partie de plus petit diamètre 26 du trou 24 de la tête épiphysaire.

Avec cette disposition, pour séparer complètement la tête épiphysaire de la tige anatomique, il faut dévisser la vis. Pour mettre en place la vis, il faut d'abord la visser dans la partie 26A filetée du trou de la tête épiphysaire. Ceci à l'avantage de rendre la vis solidaire de cette tête épiphysaire tout en la laissant libre en rotation et en translation sur une certaine longueur et ainsi facilite les manipulations par le chirurgien qui met en place la prothèse.

La surface polaire 22 comporte une pluralité d'encoches 28 disposées de façon radiales par rapport à l'axe du plot cylindrique 23 et à une distance de ce plot telle que, lorsque le plot cylindrique 23 est disposé à l'intérieur du trou 8 de la tige anatomique 1, le plot 11 situé sur la surface 7 de l'extrémité proximale de la tige anatomique 1, puisse coopérer avec une encoche 28. Ces encoches sont disposées en éventail de 10° en 10° et sont complétées par des repères qui permettent de déterminer la position de la tête épiphysaire 2 par rapport à la tige anatomique 1 lorsque la tête épiphysaire est disposée sur la tige anatomique et que le plot 11 est à l'intérieur d'une encoche 28.

En outre, le plan qui défini la surface polaire 22 est choisi afin que le diamètre de cette surface polaire soit suffisante pour que, quelle que soit l'orientation de la tête éphiphysaire par rapport à la tige anatomique, la surface polaire 22 s'étende latéralement au delà de la surface 7 de l'extrémité proximale de la tige anatomique. De la sorte, lorsque la prothèse est en place dans un humérus, l'os qui repousse ne forme pas de bourrelets venant au dessus de l'extrémité proximale de la tige anatomique et ainsi n'empêche pas d'extraire la prothèse

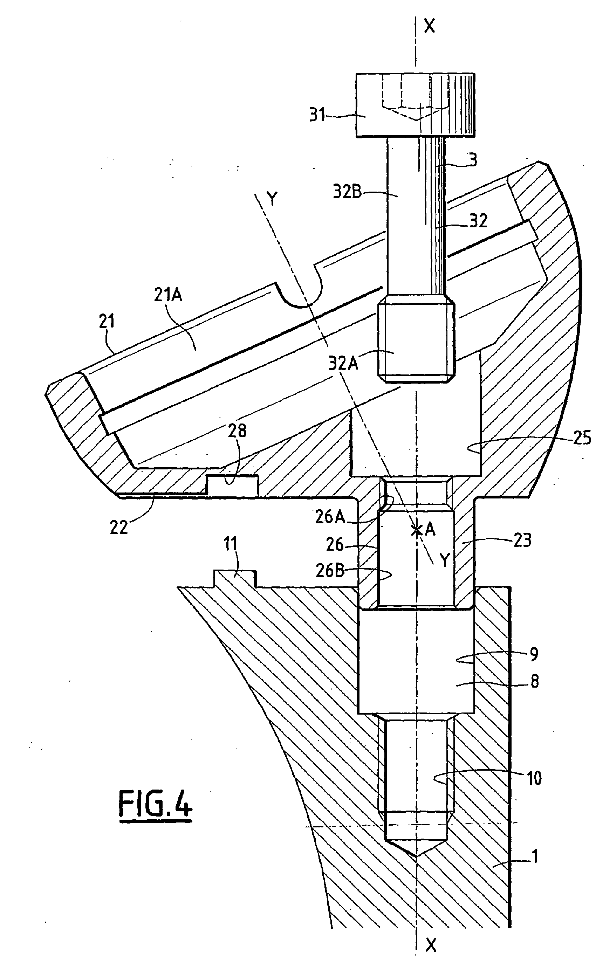

Comme on le voit sur la figure 4, la tête épiphysaire 2 peut tourner autour de l'axe longitudinal XX de la tige anatomique. L'axe YY de la tête épiphysaire, perpendiculaire au plan équatorial 21, coupe l'axe longitudinal XX de la tige anatomique 1, en un point A, de préférence situé sur la surface définie par le contact de la face proximale 7 de la tige anatomique 1 et la face polaire 22 de la tête épiphysaire 2.

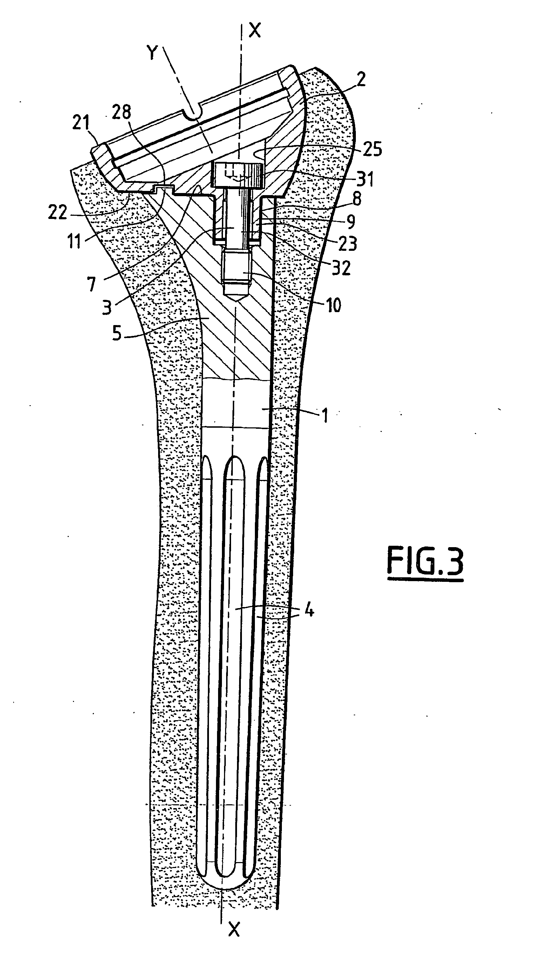

Enfin, figure 3, on voit que la longueur de la tige anatomique et les dimensions de la tête épiphysaire sont choisies pour que, lorsque la prothèse est en place, la tête épiphysaire est entièrement incluse dans l'épiphyse de l'humérus.

Pour mettre en place une telle prothèse, le chirurgien commence par préparer l'humérus en réalisant, de façon connue, un trou axial adapté pour recevoir une tige humérale anatomique et une tête épiphysaire. Puis, à l'aide d'un calibre adapté, il détermine l'angle de rétroversion que devra faire la tête épiphysaire par rapport à la tige humérale anatomique.

Le chirurgien met alors en place la tige -anatomique -puis la tête épiphysaire en l'orientant selon l'angle déterminé précédent et l'immobilise en rotation en faisant coopérer le plot 11 de l'extrémité proximale de la tige anatomique avec la rainure 28 adaptée de la surface polaire 22 de la tête épiphysaire. Enfin, il serre la vis 3 afin de bloquer l'ensemble.

## Revendications

1. Prothèse humérale modulaire du type comprenant une tige anatomique (1) et une tête épiphysaire (2) séparable pouvant être orientée angulairement par rotation autour de l'axe longitudinal (XX) de la tige anatomique, dans laquelle la tige anatomique (1) et la tête épiphysaire (2) comportent des moyens complémentaires (8,9, 11, 23, 28) de centrage, de guidage en rotation, d'indexation angulaire et de blocage en rotation de l'un par rapport à l'autre, et pour rendre solidaire la tête épiphysaire (2) et la tige anatomique (1), les moyens complémentaires comprenant une vis (3) qui peut passer à travers des trous alignés (8, 24) prévus dans la tête éphiphysaire (2) et dans la tige anatomique (1) et qui s'étendent dans l'axe longitudinal de la tige (1) pour connecter la tête épiphysaire (2) à la tige (1) **caractérisé en ce que** :
a) la tête épiphysaire (2) comprend une cuvette (21A) pour recevoir une cupule d'une prothèse d'épaule inversée coopérant avec une prothèse d'omoplate;
b) la vis (3) a une extrémité filetée (32A) et un fût (32B) entre la tête (31) et l'extrémité filetée (32A) ;
c) le trou (24) dans la tête epiphysaire (2) destiné à recevoir la vis (3) est fileté de sorte que l'extrémité filetée (32A) de la vis (3) peut y être vissée et le fût (32B) peut y coulisser, et
d) le trou (24) dans la tête épiphysaire (2) s'ouvre dans la cuvette (21A) de sorte que la tête de la vis (3) est située dans la cuvente (21A) lorsque la vis (3) connecte la tête épiphysaire (2) la tige (1)

2. Prothèse humérale modulaire selon la revendication 1, **caractérisée en ce que** les moyens complémentaires d'indexage et de blocage en rotation sont d'une part une pluralité d'encoches (28) prévues dans une surface (22) de contact de la tête épiphysaire (2) avec la tige anatomique (1), disposées selon une répartition angulaire radiale autour de l'axe longitudinal (XX) de la tige anatomique, et d'autre part un plot (11) porté par l'autre surface (7) de contact de la tige anatomique (1) avec la tête épiphysaire (2) et pouvant coopérer avec lesdites encoches.

3. Prothèse humérale modulaire selon la revendication 1 ou la revendication 2, **caractérisée en ce que** la tige anatomique (1) comprend au moins une nervure (6) longitudinale de blocage en rotation.

4. Prothèse humérale modulaire selon l'une quelconque des revendications 1 à 3, **caractérisée en ce que** la jonction entre la tige anatomique (1) et la tête épiphysaire (2) est prévue pour se situer à l'intérieur de l'humérus lorsque la prothèse est en place, afin de respecter la tension du deltoïde.

5. Prothèse humérale modulaire selon la revendication 4, **caractérisée en ce que** la surface de contact (22) de la tête épiphysaire (2) s'étend latéralement au-delà de la tige anatomique (1) quelque soit l'orientation angulaire de celle-ci.

## Claims

1. A modular humeral prosthesis of the type comprising an anatomical stem (1) and a separable epiphyseal head (2) which may be oriented angularly by rotation about the longitudinal axis (XX) of the anatomical stem, in which the anatomical stem (1) and the epiphyseal head (2) comprise complementary means (8, 9, 11, 23, 28) for centring, rotational guidance, angular indexation and rotational locking of the one relative to the other and, to connect the epiphyseal head (2) and the anatomical stem (1) firmly to one another, complementary means comprising a screw (3) which may pass through aligned holes (8, 24) provided in the epiphyseal head (2) and in the anatomical stem (1) and which extend along the longitudinal axis of the stem (1) to connect the epiphyseal head (2) to the stem (1), **characterised in that**:
a) the epiphyseal head (2) comprises a socket (21A) for receiving a cup of a reverse shoulder prosthesis cooperating with a scapular prosthesis;
b) the screw (3) has a threaded end (32A) and a shaft (32B) between the head (31) and the threaded end (32A);
c) the hole (24) in the epiphyseal head (2), intended to receive the screw (3), is threaded such that the threaded end (32A) of the screw (3) may be screwed therein and the shaft (32B) may slide therein, and
d) the hole (24) in the epiphyseal head (2) opens into the socket (21A) such that the head of the screw (3) is situated in the socket (21A) when the screw (3) connects the epiphyseal head (2) and the stem (1).

2. A modular humeral prosthesis according to claim 1, **characterised in that** the complementary indexing and rotational locking means are on the one hand a plurality of notches (28) provided in one contact surface (22) of the epiphyseal head (2) with the anatomical stem (1), disposed according to a radial angular distribution about the longitudinal axis (XX) of the humeral stem, and on the other hand a peg (11) borne by the other contact surface (7) of the anatomical stem (1) with the epiphyseal head (2) and capable of cooperating with said notches.

3. A modular humeral prosthesis according to claim 1 or claim 2, **characterised in that** the anatomical stem (1) comprises at least one longitudinal rib (6) for rotational locking.

4. A modular humeral prosthesis according to any one of claims 1 to 3, **characterised in that** the junction between the anatomical stem (1) and the epiphyseal head (2) is intended to be located inside the humerus when the prosthesis is in place, in order to take account of deltoid tension.

5. A modular humeral prosthesis according to claim 4, **characterised in that** the contact surface (22) of the epiphyseal head (2) extends laterally beyond the anatomical stem (1) whatever the angular orientation of the latter.

## Patentansprüche

1. Modulare Oberarmprothese des Typs, der einen anatomischen Schaft (1) und einen trennbaren, durch Rotation um die Längsachse (XX) des anatomischen Schafts winkelmäßig orientierbaren epiphysischen Kopf (2) umfasst und bei der der anatomische Schaft (1) und der epiphysische Kopf (2) komplementäre Einrichtungen (8, 9, 11, 23, 28) zur Zentrierung, Rotationsführung, Winkelindexierung und gegenseitigen Rotationsblockierung umfasst, wobei die komplementären Einrichtungen zur Verbindung des epiphysischen Kopfs (2) und des anatomischen Schafts (1) eine Schraube (3) umfassen, die fluchtende Löcher (8, 24), die in dem epiphysischen Kopf (2) und dem anatomischen Schaft (1) vorgesehen sind und sich gemäß der Längsachse des Schafts (1) erstrecken, durchqueren kann, um den epiphysischen Kopf (2) mit dem anatomischen Schaft (1) zu verbinden,
**dadurch gekennzeichnet:**
a) **dass** der epiphysische Kopf (2) eine Vertiefung (21A) zur Aufnahme einer Gelenkpfanne einer mit einer Schulterblattprothese kooperierenden inversen Schulterprothese umfasst;
b) **dass** die Schraube (3) ein gewindetes Ende (32A) und einen Schaft (32B) zwischen dem Kopf (31) und dem gewindeten Ende (32A) hat;
c) **dass** das Loch (24) in dem epiphysischen Kopf (2), bestimmt zur Aufnahme der Schraube (3), so gewindet ist, dass das gewindete Ende (32A) der Schraube (3) dort geschraubt werden kann und der Schaft (32B) dort gleiten kann, und
d) **dass** sich das Loch (24) in dem epiphysischen Kopf (2) in der Vertiefung (21A) so öffnet, dass der Kopf der Schraube (3) sich in der Vertiefung (21A) befindet, wenn die Schraube (3) den epiphysischen Kopf (3) und die Stange (1) verbindet.

2. Modulare Oberarmprothese nach Anspruch 1, **dadurch gekennzeichnet, dass** die komplementären Einrichtungen zur Indexierung und Rotationsblockierung einerseits eine Vielzahl Rastkerben (28) umfassen, vorgesehen in einer Kontaktfläche (22) des epiphysischen Kopfs (2) mit dem anatomischen Schaft (1) und angeordnet gemäß einer radialen Winkeleinteilung um die Längsachse (XX) des anatomischen Schafts herum, und andererseits einen Stift (11) umfassen, den die andere Kontaktfläche (7), des anatomischen Schafts (1) mit dem epiphysischen Kopf (2), trägt und der mit den genannten Rastkerben kooperieren kann.

3. Modulare Oberarmprothese nach Anspruch 1 oder Anspruch 2, **dadurch gekennzeichnet, dass** der anatomische Schaft (1) wenigstens eine longitudinale Rotationsblockierungsrippe (6) umfasst.

4. Modulare Oberarmprothese nach einem der Ansprüche 1 bis 3, **dadurch gekennzeichnet, dass** die Schnittstelle zwischen dem anatomischen Schaft (1) und dem epiphysischen Kopf (2) so vorgesehen ist, dass sie sich im Innern des Oberarmknochens befindet, wenn die Prothese eingesetzt ist, um der Spannung des Deltamuskels Rechnung zu tragen.

5. Modulare Oberarmprothese nach Anspruch 4, **dadurch gekennzeichnet, dass** die Kontaktfläche (22) des epiphysischen Kopfs (2) sich seitlich über den anatomischen Schaft (1) hinaus erstreckt, unabhängig von dessen Winkelorientierung.
